(19) Europäisches Patentamt / European Patent Office / Office européen des brevets

(11) **EP 3 215 004 B1**

(12) **EUROPEAN PATENT SPECIFICATION**

(45) Date of publication and mention of the grant of the patent:
**08.06.2022 Bulletin 2022/23**

(21) Application number: **15793727.7**

(22) Date of filing: **28.10.2015**

(51) International Patent Classification (IPC):
*A61B 5/01* (2006.01)    *A61B 5/00* (2006.01)

(52) Cooperative Patent Classification (CPC):
**A61B 5/01; A61B 5/4848**

(86) International application number:
**PCT/EP2015/075008**

(87) International publication number:
**WO 2016/071180 (12.05.2016 Gazette 2016/19)**

(54) **METHODS USING FACIAL TEMPERATURE TO EVALUATE POST-INGESTIVE IMPACT OF FOOD INGREDIENTS ON FAT OXIDATION**

VERFAHREN MIT VERWENDUNG DER GESICHTSTEMPERATUR ZUR AUSWERTUNG DER AUSWIRKUNGEN VON NAHRUNGSMITTELBESTANDTEILEN AUF DIE FETTOXIDATION NACH DER EINNAHME

PROCÉDÉS D'UTILISATION DE LA TEMPÉRATURE FACIALE POUR ÉVALUER L'IMPACT POST-INGESTION D'INGRÉDIENT ALIMENTAIRES SUR L'OXYDATION DES GRAISSES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.11.2014 US 201462074383 P**

(43) Date of publication of application:
**13.09.2017 Bulletin 2017/37**

(73) Proprietor: **Société des Produits Nestlé S.A.**
**1800 Vevey (CH)**

(72) Inventors:
• **MICHLIG GONZALEZ, Stéphanie**
  **CH-1052 Le Mont-sur-Lausanne (CH)**
• **BEAUMONT, Maurice**
  **CH-1610 Oron La Ville (CH)**
• **LE COUTRE, Johannes**
  **CH-1009 Pully (CH)**

(74) Representative: **Kamibayashi, Lynne**
**Société des Produits Nestlé S.A.**
**Avenue Nestlé 55**
**1800 Vevey (CH)**

(56) References cited:
**WO-A1-01/35819**

• A. T. CLARK, J. S. MANGAT, S. S. TAY, Y. KING, C. J. MONK, P. A. WHITE, P. W. EWAN: "Facial thermography is a sensitive and specific method for assessing food challenge outcome", ALLERGY, vol. 62, no. 7, July 2007 (2007-07), pages 744-749, XP002752516, DOI: 10.1111/j.1398-9995.2007.01363.x
• ClinicalTrials.gov: "Physiologic Effect of Spices Ingestion", , no. NCT02193438 16 July 2014 (2014-07-16), XP002752517, Retrieved from the Internet: URL:https://clinicaltrials.gov/show/NCT021 93438 [retrieved on 2015-12-22]

**Description**

BACKGROUND

The present disclosure generally relates to methods evaluating and/or predicting the post-ingestive impact of food ingredients on fat oxidation. More specifically, the present disclosure relates to use facial thermography (or any other device) to identify ingredients that could improve fat oxidation by measuring their capacity to induce an increase of nose temperature.

Research on the molecular mechanisms underlying pungent sensations revealed the existence of two cation channels, TRPV1 (transient receptor potential VI) and TRPA1 (transient receptor potential A1) that are expressed in the somatosensory fibers innervating the oral cavity. TRPV1 is the receptor for heat and burning sensations such as capsaicin, the spicy compound of chili peppers. TRPA1 responds to cold and pungent compounds.

During the past decades, the prevalence of obesity has increased worldwide to epidemic proportion. Approximately 1 billion of people worldwide are overweight or obese, conditions that increase mortality, mobility and economical costs. Obesity develops when energy intake is greater than energy expenditure, the excess energy being stored mainly as fat in adipose tissue. Body weight loss and prevention of weight gain can be achieved by reducing energy intake or bioavailability, increasing energy expenditure, and/or reducing storage as fat.

The TRPV1 agonist capsaicin is well known as increasing energy expenditure and fat oxidation, but the efficient doses are intermediate to high (20 mg and more). See, e.g., Ludy et al, "The effects of hedonically acceptable red pepper doses on thermogenesis and appetite," Physiol. Behav., Mar. 1, 102(3-4): 251-8 (2011). Moreover, capsaicin is a particularly pungent and toxic compound. Physiological effects associated with oral administration of capsaicin include a burning sensation of heat from the mid-tongue to the throat, shortness of breath, fainting, nausea, and spontaneous vomiting. As a result, only small quantities of capsaicin may be administered without causing discomfort to the individual. Food products containing capsaicin are frequently not accepted by the consumer because such products provide a very unpleasant mouth feeling. In particular, the burning effects are considered to be very unsavory, affecting the consumption of the food product.

So far, the only spice-derived ingredient showing an impact on human metabolism is capsaicin. For example, a study that looked at the effect of mustard, horseradish, black pepper and ginger on energy balance and food intake in humans did not identify any effect of these raw spices. Gregersen et al., "Acute effects of mustard, horseradish, black pepper and ginger on energy expenditure, appetite, ad libitum energy intake and energy balance in human subjects," Br. J. Nutr., 5:1-8 (July 2012). However, the effective dosage of capsaicin is too intense to be included in a food product, due to spicy taste, or to be ingested, due to gastrointestinal intolerance. A. T. CLARK, J. S. MANGAT, S. S. TAY, Y. KING, C. J. MONK, P. A. WHITE, P. W. EWAN, "Facial thermography is a sensitive and specific method for assessing food challenge outcome", ALLERGY, vol. 62, no. 7, July 2007 (2007-07), pages 744-749, DOI: 10.1111/j.1398-9995.2007.01363.x describes facial thermography as an aid detecting allergic reaction to a food allergen.

SUMMARY

The present inventors surprisingly discovered that using facial thermography would allow evaluation and prediction of the post-ingestive impact of food ingredients on fat oxidation by tracking changes on nose temperature. Therefore, methods are disclosed herein to evaluate fat oxidation after the ingestion of specific ingredients or in postprandial condition, compared to a reference.

Accordingly, in a general embodiment, the present disclosure provides a method comprising: using a thermography device to measure a first temperature of a region of a face of a subject before the subject ingests a test compound; using the thermography device to measure a second temperature of the region of the face of the subject after the test compound is ingested; and classifying the test compound as a compound that stimulates fat oxidation or a compound that does not stimulate fat oxidation based at least partially on a difference between the second temperature and the first temperature.

In an embodiment, the region comprises a nose of the subject.

In an embodiment, the thermography device is an infrared camera.

In an embodiment, the first temperature is part of a first plurality of temperatures obtained in first predetermined intervals over a first predetermined time period before ingestion of the test compound by the subject.

In an embodiment, the second temperature is part of a second plurality of temperatures obtained in second predetermined intervals over a second predetermined time period after ingestion of the test compound by the subject, and the classifying of the test compound is based at least partially on a difference between each of the second plurality of temperatures and the first temperature. The second predetermined time period can begin at the ingestion of the test compound by the subject and can end at least fifteen minutes after the administering of the test compound. The second predetermined intervals can be about one second apart from each other. The method can comprise determining, for at least one of the second plurality of temperatures, an area under the curve (AUC) based on the difference as a function of time, wherein the classifying of the test compound is based at least partially on the AUC.

In an embodiment, the first temperature is part of a first plurality of temperatures obtained in first predetermined intervals over a first predetermined time period before ingestion of the test compound by the subject, the second temperature is part of a second plurality of temperatures obtained in second predetermined intervals over a second predetermined time period after the ingestion of the test compound by the subject, and the classifying of the test compound is based at least partially on a difference between each of the second plurality of temperatures and an average of the first plurality of temperatures. The method can comprise determining, for at least one of the second plurality of temperatures, an area under the curve (AUC) based on the difference as a function of time, wherein the classifying of the test compound is based at least partially on the AUC.

In an embodiment, the method comprises administering the test compound to the subject.

In an embodiment, the test compound is classified without using indirect calorimetry.

In another embodiment, the present disclosure provides a method comprising: measuring a temperature change in a region of a face of a subject that has ingested a compound; determining whether the compound stimulates fat oxidation based at least partially on the temperature change; and incorporating the compound into a food product if the compound stimulates fat oxidation.

In an embodiment, the food product is selected from the group consisting of beverages, baked products, cereal bars, snack-foods, soups, breakfast cereals, muesli, candies, tabs, cookies, biscuits, crackers, and dairy products.

In an embodiment, the temperature change is measured at predetermined time intervals for at least fifteen minutes after ingestion of the compound by the subject. In other embodiments, the temperature change is measured at predetermined time intervals for less than fifteen minutes after ingestion of the compound by the subject. The predetermined time intervals can comprise at least one of (i) five minutes after ingestion, (ii) ten minutes after ingestion, or (iii) fifteen minutes after ingestion, preferably all three of these time points.

In another embodiment, the present disclosure provides a method comprising: measuring a temperature change in a region of a face of a subject before the subject ingests a test compound; determining whether the test compound stimulates fat oxidation based at least partially on the temperature change; and if the compound stimulates fat oxidation, administering the compound to an individual in a therapeutically effective amount to promote weight maintenance or weight loss in the individual.

In an embodiment, the individual is on a weight loss program.

An advantage of the present disclosure is to identify food ingredients that increase energy expenditure.

Another advantage of the present disclosure is to identify food ingredients that increase sympathetic nervous system activity.

Still another advantage of the present disclosure is to identify food ingredients that increase fat oxidation.

Yet another advantage of the present disclosure is to identify compounds that can be easily and safely used in food products and also increase energy expenditure, sympathetic nervous system activity, and fat oxidation.

An additional advantage of the present disclosure is to identify naturally-occurring compounds that increase energy expenditure, sympathetic nervous system activity, and fat oxidation.

Another advantage of the present disclosure is to identify food ingredients that increase energy expenditure, sympathetic nervous system activity, and fat oxidation with tolerable side effects or no side effects.

Yet another advantage of the present disclosure is to identify food ingredients that support weight management, promote weight loss, and/or treat or prevent obesity or overweight.

A further advantage of the present disclosure is to measure substrate oxidation without using indirect calorimetry, for example without measuring $O_2$ consumption and $CO_2$ production using a canopy or a metabolic sarcophagi.

An additional advantage of the present disclosure is a method for measuring fat oxidation that is non-invasive and non-intrusive and limits the intervention and contact with investigated subjects.

Additional features and advantages are described herein, and will be apparent from, the following Detailed Description and the Figures.

BRIEF DESCRIPTION OF THE FIGURES

FIG. 1A is a schematic diagram of the study conducted in the experimental example.

FIG. 1B shows a graphical user interface (GUI) in which regions of interest on a subject's face were defined in the experimental example.

FIG. 2 is a graph of binding as a function of concentration for Givaudan, used as a control in the experimental example.

FIG. 3A is a graph of energy expenditure results from the experimental example.

FIG. 3B is a graph of fat oxidation results from the experimental example.

**FIG. 3C** is a graph of carbohydrate oxidation results from the experimental example.
**FIG. 3D** is a graph of time standardized energy expenditure results in the experimental example.
**FIG. 3E** is a graph of time standardized fat oxidation results from the experimental example.
**FIG. 3F** is a graph of time standardized carbohydrate oxidation results from the experimental example.
**FIG. 4A** is a graph of the change in nose temperature relative to placebo after intake in the experimental example.
**FIG. 4B** is a graph of the change in cheek temperature relative to placebo after intake in the experimental example.

DETAILED DESCRIPTION

All percentages expressed herein are by weight of the total weight of the composition unless expressed otherwise. When reference is made to the pH, values correspond to pH measured at 25 °C with standard equipment. As used in this disclosure and the appended claims, the singular forms "a," "an" and "the" include plural referents unless the context clearly dictates otherwise. The term "and/or" used in the context of "X and/or Y" should be interpreted as "X," or "Y," or "X and Y."

As used herein, "about" is understood to refer to numbers in a range of numerals, for example the range of -10% to +10% of the referenced number, preferably within -5% to +5% of the referenced number, more preferably within -1% to +1% of the referenced number, most preferably within -0.1% to +0.1% of the referenced number. Furthermore, all numerical ranges herein should be understood to include all integers, whole or fractions, within the range. Moreover, these numerical ranges should be construed as providing support for a claim directed to any number or subset of numbers in that range. For example, a disclosure of from 1 to 10 should be construed as supporting a range of from 1 to 8, from 3 to 7, from 1 to 9, from 3.6 to 4.6, from 3.5 to 9.9, and so forth.

The compositions disclosed herein may lack any element that is not specifically disclosed herein. Thus, a disclosure of an embodiment using the term "comprising" includes a disclosure of embodiments "consisting essentially of" and "consisting of" the components identified.

"Prevention" includes reduction of risk and/or severity of a condition or disorder. The terms "treatment," "treat" and "to alleviate" include both prophylactic or preventive treatment (that prevent and/or slow the development of a targeted pathologic condition or disorder) and curative, therapeutic or disease-modifying treatment, including therapeutic measures that cure, slow down, lessen symptoms of, and/or halt progression of a diagnosed pathologic condition or disorder; and treatment of patients at risk of contracting a disease or suspected to have contracted a disease, as well as patients who are ill or have been diagnosed as suffering from a disease or medical condition. The term does not necessarily imply that a subject is treated until total recovery. The terms "treatment" and "treat" also refer to the maintenance and/or promotion of health in an individual not suffering from a disease but who may be susceptible to the development of an unhealthy condition. The terms "treatment," "treat" and "to alleviate" are also intended to include the potentiation or otherwise enhancement of one or more primary prophylactic or therapeutic measure. The terms "treatment," "treat" and "to alleviate" are further intended to include the dietary management of a disease or condition or the dietary management for prophylaxis or prevention a disease or condition. A treatment can be patient- or doctor-related.

The terms "food," "food product" and "food composition" mean a product or composition that is intended for ingestion by a human and provides at least one nutrient to the human.

"Overweight" is defined for a human as a BMI between 25 and 30. "Obese" is defined for a human as a BMI greater than 30. "Weight loss" is a reduction of the total body weight. Weight loss may, for example, refer to the loss of total body mass in an effort to improve fitness, health, and/or appearance. "Weight management" or "weight maintenance" relates to maintaining a total body weight. For example, weight management may relate to maintaining a BMI in the area of 18.5-25 which is considered to be normal.

A "low-fat" diet is a diet with less than 20% of the calories from fat, preferably less than 15% from fat. A "low-carbohydrate" diet is a diet with less than 20% of the calories from carbohydrates. A "low-calorie diet" is a diet with less calories per day relative to the individual's previous intake before the diet and/or a diet with less calories per day relative to an average person of similar body type. A "very low-calorie" diet is a diet with 800 kcal (3,300 kJ) per day or less.

As used herein, an "effective amount" is an amount that prevents a deficiency, treats a disease or medical condition in an individual or, more generally, reduces symptoms, manages progression of the diseases or provides a nutritional, physiological, or medical benefit to the individual.

"Animal" includes, but is not limited to, mammals, which includes but is not limited to, rodents, aquatic mammals, domestic animals such as dogs and cats, farm animals such as sheep, pigs, cows and horses, and humans. Where "animal," "mammal" or a plural thereof is used, these terms also apply to any animal that is capable of the effect exhibited or intended to be exhibited by the context of the passage. As used herein, the term "patient" is understood to include an animal, especially a mammal, and more especially a human that is receiving or intended to receive treatment, as treatment is herein defined. While the terms "individual" and "patient" are often used herein to refer to a human, the present disclosure is not so limited. Accordingly, the terms "individual" and "patient" refer to any animal, mammal or human, that can benefit from the treatment.

The methods disclosed herein are based on the measurement of the modification of nose temperature using an infrared camera and/or another thermo imaging device. The present inventors showed a correlation between the impact of ingredients on the nose temperature and the capacity of the ingredients to maintain a higher post-ingestive fat oxidation. Without being bound by theory, the present inventors propose that facial thermography (or any other device) can be used to identify ingredients that could improve fat oxidation by measuring their capacity to induce an increase of nose temperature. The inventors believe that the higher fat oxidation observed with the ingredients tested in the clinical trial disclosed later herein is associated with a thermogenic phenomenon. The increase in nose temperature would reflect the dissipation of temperature by vasodilatation of capillaries in response to the thermogenesis induced by the ingestion of the ingredients. Subjects can be continuously recorded using an infrared camera during ingestion of the compounds. As discussed in the section of this application directed to the clinical trial, the thermography data showed a good correlation with the effect measured in parallel via indirect calorimetry.

Specifically, an exploratory clinical trial was conducted to evaluate the impact of spicy ingredient ingestion on metabolism and the activity of the autonomic nervous system. Facial thermography using an infrared camera was explored as a tool to capture changes of temperature on the face induced by modification of the activity of the autonomic nervous system. In this study, the present inventors observed a correlation between changes of the nose temperature (an increase) and recorded levels of fat oxidation after ingestion of some ingredients. Therefore, the present disclosure provides methods comprising using facial thermography to measure the impact of a food ingredient on metabolism and more precisely on fat oxidation after consumption.

Accordingly, an aspect of the present disclosure is a method of identifying a compound that stimulates energy expenditure and/or fat oxidization. A pre-ingestion facial temperature reading can be generated in which a temperature of at least one region of the face of a subject is measured by a thermography device. For example, the temperature of the at least one region of the face of the subject can be measured in first predetermined intervals over a first predetermined time period. In an embodiment, the thermography device is an infrared thermography camera. However, the thermography device can be any device that detects radiation in the infrared range of the electromagnetic spectrum (9-14 $\mu$m) and produces images of the detected radiation ("thermograms"), and the present disclosure is not limited to a specific embodiment of the thermography device. The thermograms can be used to determine the temperatures, for example using an algorithm executed by a processor of the thermography device and/or executed by an apparatus associated with the thermography device.

Non-limiting examples of suitable first predetermined intervals include measuring the temperature of the at least one region of the face every second, every ten seconds, every minute, or the like. Non-limiting examples of suitable first predetermined time periods include the thirty minutes before ingestion of the compound, the twenty minutes before ingestion, the ten minutes before ingestion, one minute before ingestion, or the like. In a preferred embodiment, a baseline temperature is calculated from the temperature of the at least one region of the face measured in the first predetermined intervals over the first predetermined time period. Alternatively, a baseline temperature can be a single measurement, although this embodiment is less preferred.

After the first predetermined time period, a test compound is ingested by the subject. In an embodiment, the method comprises administering the test compound to the subject. The test compound can be any compound safe for ingestion, such as naturally occurring food ingredients or compounds found therein. For example, the test compound can be transient receptor potential (TRP) agonist. The test compound can be administered in a beverage and/or another edible composition. Preferably, the test compound is ingested without another compound that affects fat oxidation i.e. increases or decreases fat oxidation.

After ingestion, a post-ingestion facial temperature reading can be generated in which a temperature of the at least one region of the face of the subject is measured by the thermography device. For example, the temperature of the at least one region of the face of the subject can be measured in second predetermined intervals over a second predetermined time period. Preferably a constant distance is maintained between the thermography device and the face throughout the entirety of the first and second predetermined time periods. The test compound preferably is ingested in a single dose and without any other compositions ingested during the first and second predetermined time periods, most preferably after an all-night fast.

Non-limiting examples of suitable second predetermined intervals include measuring the temperature of the at least one region of the face every second, every ten seconds, every minute, or the like. Non-limiting examples of suitable second predetermined time periods include at least one-hundred twenty minutes after ingestion of the compound, at least ninety minutes after ingestion, at least sixty minutes after ingestion, at least thirty minutes after ingestion, at least fifteen minutes after ingestion, at least ten minutes after ingestion, at least five minutes after ingestion, or the like. In a preferred embodiment, the post-ingestion temperatures are compared to the baseline temperature to calculate changes in temperatures.

The skilled artisan will readily recognize how to further refine the methods to achieve a desired degree of statistical certainty. In this regard, the methods explicitly disclosed herein will have many variants that can be implemented by the skilled artisan and which are encompassed by the present disclosure.

In an embodiment, the identified compound can be used in a method to support weight management or promote weight loss. For example, the identified compound can be incorporated into a composition administered to a mammal managing their weight or undergoing a weight loss program, such as a weight loss diet (e.g., one or more of a low-fat diet, a low-carbohydrate diet, a low-calorie diet and a very low-calorie diet) and/or a weight loss training regimen (e.g. endurance and/or strength training). In another embodiment, the identified compound can be incorporated into a composition used in a method for preventing obesity or overweight by administering the composition to a mammal at risk thereof. In yet another embodiment, the identified compound can be incorporated into a composition used in a method for treating obesity or overweight by administering the composition to a mammal in need thereof. In an embodiment, the identified compound can be incorporated into a composition administered to a human. The composition comprising the identified compound can be administered in a single dose, although multiple doses are also encompassed by the present disclosure. The composition can also comprise an additional weight loss ingredient.

The composition comprising the identified compound may be a medicament, a food product, a medical food, an oral nutritional supplement, a nutritional composition, an oral cosmetics or a supplement to a food product and is preferably orally administered, most preferably as a beverage. A medical food product is specially formulated and intended for the dietary management of diseases or medical conditions (e.g., prevent or treat diseases or undesirable medical conditions). A medical food product can provide clinical nutrition, for example fulfilling special nutritional needs of patients with a medical condition or other persons with specific nutritional needs. A medical food product can be in the form of a complete meal, part of a meal, as a food additive, or a powder for dissolution.

A food product, medical food or nutritional composition includes any number of optional additional ingredients, including conventional food additives, for example one or more proteins, carbohydrates, fats, acidulants, thickeners, buffers or agents for pH adjustment, chelating agents, colorants, emulsifiers, excipients, flavor agents, minerals, osmotic agents, a pharmaceutically acceptable carrier, preservatives, stabilizers, sugars, sweeteners, texturizers and/or vitamins. The optional ingredients can be added in any suitable amount.

A food product, medical food or nutritional composition can be in any oral nutritional form, e.g. as a health drink, as a ready-made drink, optionally as a soft drink, including juices, milk-shake, yogurt drink, smoothie or soy-based drink, in a bar, or dispersed in foods of any sort, such as baked products, cereal bars, dairy bars, snack-foods, soups, breakfast cereals, muesli, candies, tabs, cookies, biscuits, crackers (such as a rice crackers), and dairy products.

A supplement may be in the form of tablets, capsules, pastilles or a liquid, for example. The supplement may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins or the like), adsorbents, carriers, fillers, co-compounds, dispersing agents, wetting agents, processing aids (solvents), flowing agents, taste masking agents, weighting agents, jellifying agents and gel forming agents. The supplement may also contain conventional pharmaceutical additives and adjuvants, excipients and diluents, including, but not limited to, water, gelatin of any origin, vegetable gums, ligninsulfonate, talc, sugars, starch, gum arabic, vegetable oils, polyalkylene glycols, flavoring agents, preservatives, stabilizers, emulsifying agents, buffers, lubricants, colorants, wetting agents, fillers, and the like.

The supplement can be added in a product acceptable to the consumer as an ingestible carrier or support. Non-limiting examples of such carriers or supports are a pharmaceutical, a food composition, and a pet food composition. Non-limiting examples for food and pet food compositions are milks, yogurts, curds, cheeses, fermented milks, milk-based fermented products, fermented cereal based products, milk-based powders, human milks, preterm formulas, infant formulas, oral supplements, and tube feedings.


## EXAMPLE

The following non-limiting example presents clinical scientific data developing and supporting the concept of using facial thermography would allow evaluation and prediction of the post-ingestive impact of food ingredients on fat oxidation by tracking changes on nose temperature.

The present inventors had the hypothesis that stimulation of the TRPA1 and TRPM8 pathways could incease thermogenesis to promote energy expenditure and have an antiobesity effect. The primary objective of the clinical trial was to compare the impact on energy expenditure of a relavant agonist of each of the three TRP channel of interest. Secondary objectives were to compare their impact on substate oxidation and on the activation of the SNS measured by heart rate variability analysis and facial thermography as an exploratory method.


## Materials and Methods

Participants. Participants were healthy men with 19<BMI<25 and 60 kg minimum body weight. The age was in the 20-50 range. Participants had to like spicy food but are moderate spicy food eaters. Screening of candidates was done through a questionnaire on spicy food habits. The clinical trial we approved by a competent ethics committee (Commission cantonale (VD) d'Etique pour recherché sur l'Etre Humain) and participants gave their written informed consent. This trial was conducted according to the principles and rules laid down in the Declaration of Helsinki and its subsequent amendments.

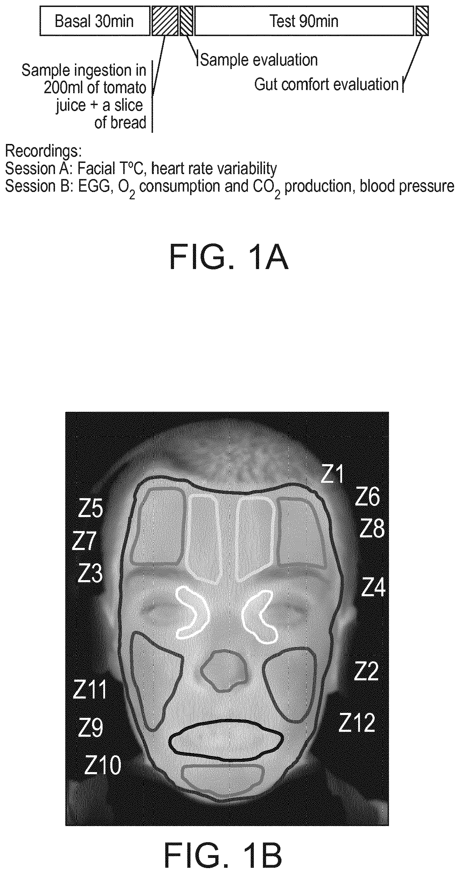

Trial design. The presented study was a double-blinded, balanced, randomized, cross-over, placebo-controlled clinical trial. For each measure, the subjects had tested after an over-night fast. They had been requested to use their car or public transportation to come to the investigation site in order to keep morning physical activity level as low as possible. Moreover, consumption of caffeine was not permitted after the lunch meal (12 p.m.) before each session, and participants had been asked to refrain their spice consumption for two days prior each session. Each sample was evaluated in two different sessions A and B, with the same design but different recordings (FIG. 1A).

Each session started at 8:00 a.m. with a 30 minutes rest period and measurement preparation. During all the testing sessions, subjects were seated comfortably. Room temperature was in the 20 to 22 °C range. Subjects were instructed to remain quiet and still, but they were allowed to watch TV. Parameters were recorded 30 minutes before and 90 minutes after ingestion of each sample (FIG. 1A).

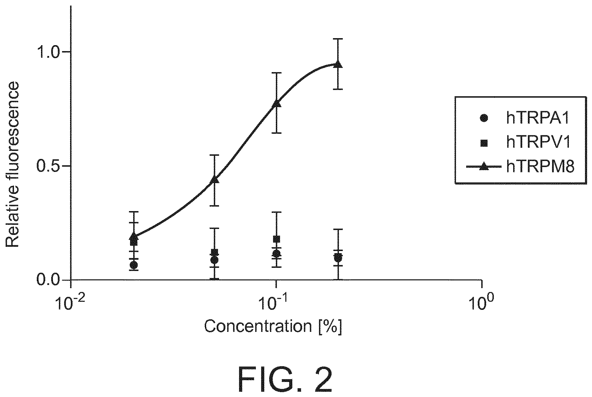

Treatment. All active TRP agonists were in 200 ml liquid tomato juice (Granini) at room temperature and served with a slice of bread. Placebo was 200 ml Commercial tomato juice. Active ingredients per 200ml were 1 mg of Capsaicin (Spectrum chemicals), 0.2 ml of Givaudan cooling flavor (QB-113-979-5) or 70 mg of Cinnamaldehyde (Sigma). All ingredients were *in vitro* validated in cell lines expressing hTRPV1, hTRPA1 or hTRPM8 according to the calcium imaging method described in Riera et al, 2009 (FIG. 2).

Indirect calorimetry. Energy expenditure was measured using indirect calorimetry. Indirect calorimetry is a measure of respiratory gas exchange (oxygen consumption and carbon dioxide production), which estimates the amount of calories "burned." This measurement was performed with the MAX-II device (Max II metabolic system, AEI technologies, Naperville, IL, USA). The equation used by the MAX-II device to evaluate energy expenditure is the simplified Weir formula:

$$REE = (3.941 \times VO_2) + (1.106 \times VCO_2),$$

where:

$$EE = \text{energy expenditure (kcal/min)}$$

$$VO_2 = \text{rate of oxygen consumption (l/min)}$$

and

$$VCO_2 = \text{rate of carbon dioxide production (l/min)}$$

After answering questions from the compliance questionnaire, participants were comfortably installed in semi-recumbent position in a reclining bed, at an angle allowing the participant to ingest the test solution without having to adjust position between metabolic rate measurement periods and treatment ingestion. A canopy was installed over participant's head. Test started ~08:30 a.m. following a stabilization period allowing $FeCO_2$ values to reach physiologic range.

Substrate oxidation (carbohydrates and fat utilization) and the respiratory quotient (RQ) were calculated using the $VO_2$ and $VCO_2$ values obtained from the MAX-II device during the same timeline than energy expenditure. $VO_2$ and $VCO_2$ values were corrected for protein oxidation. The following equations were used to calculate the amount of fat and carbohydrates oxidized (Dietary Reference Intakes for Energy, Carbohydrate, Fiber, Fat, Fatty Acids, Cholesterol, Protein, and Amino Acids (Macronutrients) (2002/2005) National Academies Press, pp. 633-645):

$$PO = (0.8 \times \text{body weight} \times 1000)/1440$$

$$NPVO_2 = VO_2 - PO \times 1.01031$$

$$NPVCO_2 = VCO_2 - PO \times 0.84361$$

$$FAT = (NVPO_2 - NVPCO_2)/(2.01494 \times (1-0.707))$$

$$CHO = (NVPCO_2 - 0.707 \times NVPO_2)/(0.82821 \times (1-0.707))$$

$$RQ = VCO_2/VO_2$$

$$PO = \text{protein oxidation (mg/min)},$$

$$NPVO_2 = \text{non protein } O_2 \text{ consumption (ml/min)},$$

$$NPVCO_2 = \text{non protein } CO_2 \text{ consumption (ml/min)},$$

$$FAT = \text{fat oxidation (mg/min)}$$

and

$$CHO = \text{carbohydrate oxidation (mg/min)}$$

$$RQ = \text{Respiratory quotient}$$

Facial thermography. In this study, infrared thermal imaging of the face has been used as an exploratory, non-invasive recording method of sympathetic nervous system activity. Using a FLIR A325 infrared camera (FLIR Systems, Inc) subjects were continuously recorded at a distance of ~1m. They were comfortably installed in a chair with the head maintained fixed on a chin-rest support, to facilitate the further image analysis. Room temperature was in the 20 to 22°C range. The camera FLIR A325 is equipped with a 25° lens, resolution is 320x240 pixels, and sensitivity is 0.05 °C and accuracy $\pm 2$ ° C. The recording was performed by acquiring 1 image/second. Thermographic imaging software, FLIR ResearchIR is used to acquire images.

Infrared Image analysis. Facial thermography data were extracted using in-home algorithms developed within the Matlab framework (The Mathworks Inc., Natick, MA, USA). First, a graphical user interface (GUI) was created to help defining manually a temperature threshold highlighting primarily subject's faces. An alignment of the face was then computed and twelve regions of interest (ROIs) drawn manually using the GUI (FIG. 1B). Face alignment/orientation correction was computed image by image by first creating a binary mask using the manually set temperature threshold. Holes in the binary mask were filled and the region with the greatest continuous area was selected. The selected region centroid was determined using the built-in Matlab region properties tool (regionprops). With the same tool, an ellipse was fitted within the region to obtain the angular orientation of the ellipse's major axis compared to the x-axis. The original image was then shifted to center the region centroid at the (0,0) coordinates and rotated at an angle aligning the major axis of the fitted ellipse to the y-axis (shift command: circshift / rotation command: imrotate). Face alignments were computed for all images of the recorded session and average temperatures within ROIs extracted. Alignment performance was checked visually over the whole recorded session and discrepancies corrected by re-setting new regions and re-extracting the data in extreme cases (e.g. if a subject showed strong forward or background head movements not captured by the alignment algorithm).

Statistical analysis. All endpoints have been analyzed using a linear mixed model to take into account the correlation between repeated measures. For energy expenditure and substrate oxidation, the raw data were first smoothed (using loess) the time-standardized AUC was calculated as the integral over the time after ingestion till the end of the experimental session divided by the length of this time interval.

Thermography data were collected every second and were averaged over 10 min time interval. AUCs for the entire post-ingestion period were then compared between groups considering a mixed model with the baseline measurement and the treatment group as fixed effects and the subject as random effect. For ECG data parameters, the session (A/B) was added as a fixed effect and an overall F-test was computed in order to compare the groups over the sessions. The p-values were adjusted for multiple comparisons according to Bonferroni.

In order to analyze fast changes in facial temperature before and after the ingestion of the experimental products, data over 1 minute were aggregated for "BEFORE", "AFTER", "AFTER-5 (minutes)", "AFTER-10 (minutes)" and "AFTER-15 (minutes)". P-values were computed using a mixed linear model while adjusted for multiplicity using Holm's method.

## Results

Ingredient selection. The objective of this trial was to assess if the stimulation of TRPA1 and TRPM8 pathways could be as efficient at impacting the energy balance as is the simulation of TRPV1 by capsaicin or capsiate. Based on the literature, we selected the most representative TRPA1 agonist: cinnamaldehyde activating specifically the mouse variant of TRPA1 with an EC50 of $60\mu M$, its specificity on the human form of the receptor has been confirmed *in vitro* (data not shown). In order to identify an efficient and specific agonist of hTRPM8, different ingredients with cooling properties have been evaluated *in vitro*. A cooling flavor (CF) provided by Givaudan, activating specifically hTRPM8 with an EC50 of 0.07% **(FIG.** 2) has been selected. As a positive control, capsaicin was evaluated in parallel to cinnamaldehyde and CF in this trial.

The objective was to administrate a safe and tolerable dose of each ingredient. Based on a safety evaluation and on informal sensory evaluation, dosage of the sample were 1 mg of capsaicin, 70 mg of cinnamaldehyde and 0.2 ml of CF (0.1%) in 200 ml of tomato juice. The doses represent concentrations of 16 $\mu M$, 2.6mM and 0.1% respectively for capsaicin.

Trial population. In total, 19 subjects, moderate spicy food eater, were randomized and 16 completed the study. The population's mean age was 32 years old with a BMI of 22.43 kg/m2.

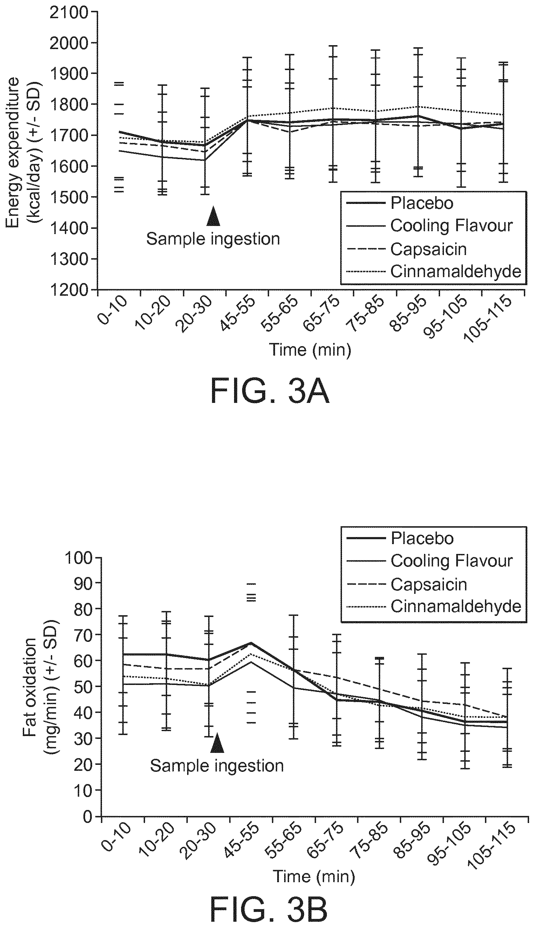

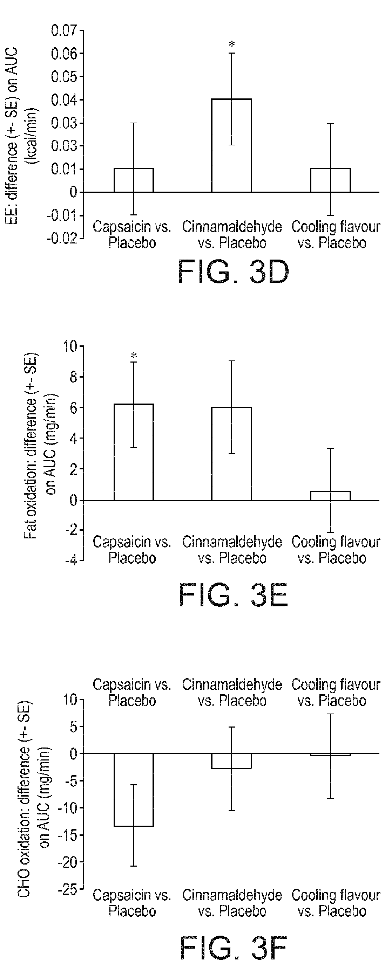

Energy expenditure. In **FIGS. 3A** and **3D,** energy expenditure results are represented as the distribution of the data averaged on 10 min intervals **(FIG. 3A)** and the difference of each treatment compared to placebo, as area under the curve (AUC) after ingestion to end of session, time standardized **(FIG. 3D).** A dose of 70 mg of cinnamaldehyde compared to placebo (unadjusted $p<0.05$) increases energy expenditure, whereas no significant differences are observed compared to placebo after the ingestion of 1 mg of capsaicin or 200 $\mu l$ of CF. Extrapolated to the 90 min of recording the difference of energy expenditure between cinnamaldehyde treatment and placebo represents 3.6 kcal.

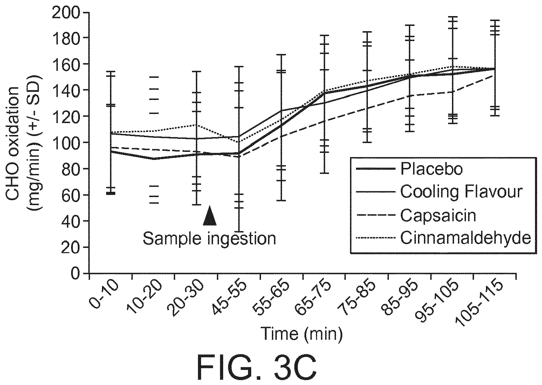

Fat and carbohydrate (CHO) oxidation. In **FIGS. 3B** and **3C,** fat and CHO oxidation results are represented as the distribution of the data averaged on 10 min intervals and the difference of each treatment compared to placebo, as area under the curve (AUC) after ingestion to end of session, time standardized **(FIGS. 3E** and **3F).** A higher level of fat oxidation is observed after the ingestion of capsaicin and cinnamaldehyde compared to placebo (unadjusted $p<0.05$). No statistically difference of fat oxidation is observed after the ingestion of the CF compared to the placebo. None of the treatment induces any significant changes of CHO oxidation compared to the placebo **(FIG. 3F).**

Facial thermography. Vasoconstriction and reduced blood flow into peripheral capillary vessels of the face as well as sweating on the forehead are phenomena that could reflect activation of the SNS under stress condition or spicy gustatory stimulation. For all products, subject's face temperature was measured every second and averaged on different zones **(FIG. 1).** AUCs were compared between groups for each zone for the all period post ingestion **(Table 1).** One significant difference could be detected in Zone-10 (chin) between cinnamaldehyde and placebo. Cinnamaldehyde increases chin temperature for a prolonged time after ingestion with an adjusted p-value of 0.0486 compared to placebo.

**Table 1.**     Comparisons of AUC of the temperature per zone

| Zone | Comparisons | AUC Difference | SE | Raw p-value | Adjusted p-value |
|---|---|---|---|---|---|
| 1 | Cap vs Pl | 18.584 | 10.377 | 0.0809 | 0.2427 |
|   | Cin vs Pl | 22.396 | 10.187 | 0.0338* | 0.1014 |
|   | CF vs Pl | 5.097 | 10.196 | 0.6198 | 1 |
| 2 | Cap vs Pl | 54.439 | 35.154 | 0.1294 | 0.3882 |
|   | Cin vs Pl | 42.064 | 34.458 | 0.2293 | 0.6879 |
|   | CF vs Pl | 24.708 | 34.551 | 0.4787 | 1 |
| 3 | Cap vs Pl | 16.959 | 13.911 | 0.2299 | 0.6897 |
|   | Cin vs Pl | 30.816 | 13.906 | 0.0324* | 0.0972 |
|   | CF vs Pl | 10.554 | 13.894 | 0.4519 | 1 |
| 4 | Cap vs Pl | 14.893 | 14.793 | 0.3201 | 0.9603 |
|   | Cin vs Pl | 22.118 | 14.637 | 0.1386 | 0.4158 |
|   | CF vs Pl | 7.862 | 14.645 | 0.5943 | 1 |
| 5 | Cap vs Pl | 8.126 | 9.672 | 0.4058 | 1 |
|   | Cin vs Pl | 14.065 | 9.562 | 0.1491 | 0.4473 |
|   | CF vs Pl | 5.246 | 9.546 | 0.5857 | 1 |
| 6 | Cap vs Pl | 13.186 | 9.899 | 0.1904 | 0.5712 |
|   | Cin vs Pl | 21.144 | 9.763 | 0.0363* | 0.1089 |
|   | CF vs Pl | 5.128 | 9.75 | 0.6018 | 1 |
| 7 | Cap vs Pl | 9.255 | 10.155 | 0.3676 | 1 |
|   | Cin vs Pl | 18.856 | 10.075 | 0.0686 | 0.2058 |
|   | CF vs Pl | 4.157 | 10.047 | 0.6813 | 1 |
| 8 | Cap vs Pl | 12.469 | 11.054 | 0.266 | 0.798 |
|   | Cin vs Pl | 18.384 | 10.881 | 0.0989 | 0.2967 |
|   | CF vs Pl | 2.828 | 10.879 | 0.7962 | 1 |
| 9 | Cap vs Pl | 26.056 | 13.146 | 0.0544 | 0.1632 |
|   | Cin vs Pl | 18.947 | 12.902 | 0.1498 | 0.4494 |
|   | CF vs Pl | 15.363 | 12.901 | 0.2407 | 0.7221 |
| 10 | Cap vs Pl | 25.26 | 13.943 | 0.0775 | 0.2325 |
|   | Cin vs Pl | 34.072 | 13.567 | **0.0162*** | **0.0486**** |
|   | CF vs Pl | 3.486 | 13.557 | 0.7984 | 1 |
| 11 | Cap vs Pl | 3.299 | 13.226 | 0.8043 | 1 |
|   | Cin vs Pl | 18.237 | 13.175 | 0.174 | 0.522 |
|   | CF vs Pl | -1.749 | 13.188 | 0.8952 | 1 |
| 12 | Cap vs Pl | 22.575 | 11.895 | 0.065 | 0.195 |
|   | Cin vs Pl | 26.571 | 11.691 | 0.0285* | 0.0855 |
|   | CF vs Pl | 9.377 | 11.693 | 0.4273 | 1 |

Abbreviations :    Cap, capsaicin; Cin, cinnamaldehyde; CF, cooling flavor.
The p-values were adjusted for multiple comparison according to Bonferroni.
* : Pvalue <0.05; ** :Adj Pvalue <0.05

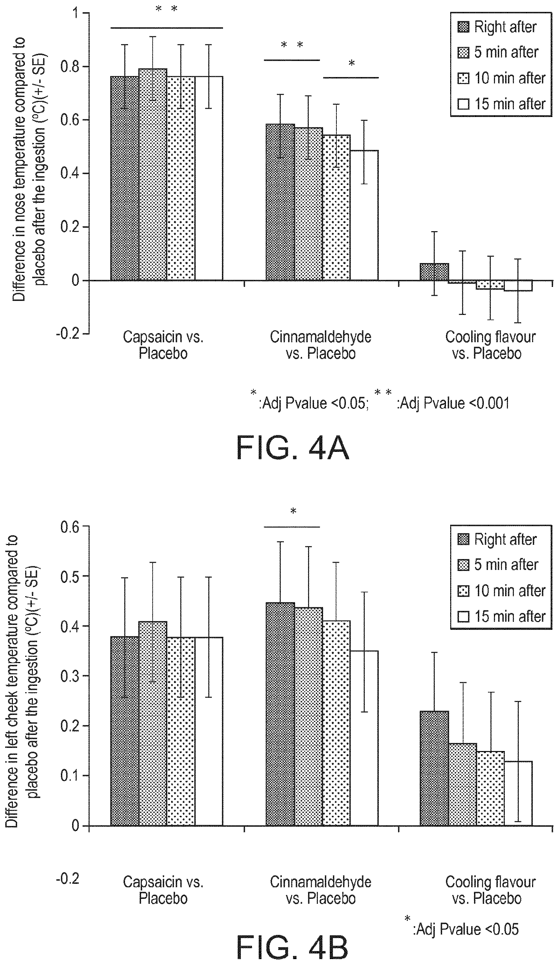

Additionally, for each zone, in order to capture fast changes of temperature induced by product ingestion, data was analyzed over the 15 minutes post-ingestion, as well as compared before and after ingestion. There is significant increase in Zone-2 (nose) after cinnamaldehyde or capsaicin intake **(FIG. 4A)** and increased temperature in Zone-12 (cheeks) immediately after intake of Cinnamaldehyde **(FIG. 4B).**

## Discussion

From the present exploratory clinical trial, it has been observed that a single ingestion of cinnamaldehyde (70mg/200ml; 350 ppm) significantly increases energy expenditure, in a magnitude of about 3.6 kcal over the period of the experiment (90 min) compared to placebo. Moreover, both capsaicin (1mg/200ml; 4.88ppm) and cinnamaldehyde maintain higher post-prandial fat oxidation, in a magnitude of about 556.2 mg and 512.7 mg, respectively, over the period of the experiment (90 min) compared to placebo. From facial thermography, cinnamaldehyde increases chin temperature in the overall treatment and both capsaicin and cinnamaldehyde increase nose temperature until 15 min after the treatment whereas only cinnamaldehyde increases unilaterally cheek temperature in the close minutes after its ingestion.

Capsaicin-induced increase of nose skin temperature is interpreted as a sympathetic vasodilator mechanism responding to thermogenesis to promote heat diffusion, as observed in rat with an increase of tail temperature. The same effect was observed after cinnamaldehyde ingestion on nose and left cheek temperature, but the opposite effect is reported on rat with cinnamaldehyde. A more sustained thermoregulatory effect on nose capillary vasodilatation at the origin of the nose temperature increase could reflect a stimulation of adrenaline secretion that could be as well at the origin of energy expenditure increase and fat oxidation. Since it has been shown in rats that intravenous administration of capsaicin or cinnamaldehyde induces adrenaline secretion, the present results suggest that both capsaicin and cinnamaldehyde induce thermogenesis via the release of adrenaline.

In the overall treatment, an increase of chin temperature was observed after cinnamaldehyde ingestion compared to placebo. This temperature increase reflects an increased blood flow in the big vessel crossing the chin. The increased blood flow could be explained by either 1) an increased cardiac output under the control of SNS stimulated by cinnamaldehyde inducing adrenaline secretion or 2) by vasodilatation of this big vessel induced by the inhibition of L-type calcium channel. Indeed it has been recently shown that cinnamaldehyde can inhibit L-type calcium channel independently of TRPA1, and induce vasorelaxation and decrease blood pressure. Even if the effect is subtle, it should not be judged as irrelevant since a cumulative approach (as dietary, exercise and behavioural) is believed to be the most efficient for sustainable weight loss or maintenance. Cinnamaldehyde could counterbalance the decrease in metabolic rate that occurs during weight loss.

It should be understood that various changes and modifications to the presently preferred embodiments described herein will be apparent to those skilled in the art. Such changes and modifications can be made without departing from the scope of the present subject matter and without diminishing its intended advantages. It is therefore intended that such changes and modifications be covered by the appended claims.

## Claims

1. A method of identifying a compound that stimulates fat oxidization, comprising:

   measuring a first temperature of a region of a face of a subject with a thermography device before the subject ingests a test compound;
   measuring a second temperature of the region of the face of the subject with the thermography device after the test compound is ingested; and
   classifying the test compound as a compound that stimulates fat oxidation or a compound that does not stimulate fat oxidation based at least partially on a difference between the second temperature and the first temperature.

2. The method of Claim 1 wherein the region comprises a nose of the subject.

3. The method of Claim 1 wherein the thermography device is an infrared camera.

4. The method of Claim 1 wherein the first temperature is part of a first plurality of temperatures obtained in first predetermined intervals over a first predetermined time period before ingestion of the test compound by the subject, and the classifying of the test compound is based at least partially on a difference between the second temperature and an average of the first plurality of temperatures.

5. The method of Claim 4 wherein the first predetermined time period begins at least thirty minutes before ingestion of the test compound by the subject and ends at the ingestion of the test compound by the subject.

6.  The method of Claim 4 wherein the first predetermined intervals are about one second apart from each other.

7.  The method of Claim 1 wherein the second temperature is part of a second plurality of temperatures obtained in second predetermined intervals over a second predetermined time period after ingestion of the test compound by the subject, and the classifying of the test compound is based at least partially on a difference between each of the second plurality of temperatures and the first temperature.

8.  The method of Claim 7 wherein the second predetermined time period begins at the ingestion of the test compound by the subject and ends at least fifteen minutes after the administering of the test compound.

9.  The method of Claim 7 wherein the second predetermined intervals are about one second apart from each other.

10. The method of Claim 7 comprising determining, for at least one of the second plurality of temperatures, an area under the curve (AUC) based on the difference as a function of time, wherein the classifying of the test compound is based at least partially on the AUC.

11. The method of Claim 1 wherein the first temperature is part of a first plurality of temperatures obtained in first predetermined intervals over a first predetermined time period before ingestion of the test compound by the subject, the second temperature is part of a second plurality of temperatures obtained in second predetermined intervals over a second predetermined time period after the ingestion of the test compound by the subject, and the classifying of the test compound is based at least partially on a difference between each of the second plurality of temperatures and an average of the first plurality of temperatures.

12. The method of Claim 11 comprising determining, for at least one of the second plurality of temperatures, an area under the curve (AUC) based on the difference as a function of time, wherein the classifying of the test compound is based at least partially on the AUC.

13. The method of Claim 1 comprising administering the test compound to the subj ect.

14. The method of Claim 1 wherein the test compound is classified without using indirect calorimetry.

15. A method comprising:

    measuring a temperature change in a region of a face of a subject that has ingested a compound, with a thermography device;
    determining whether the compound stimulates fat oxidation based at least partially on the temperature change; and
    incorporating the compound into a food product if the compound stimulates fat oxidation.

16. The method of Claim 15 wherein the food product is selected from the group consisting of beverages, baked products, cereal bars, snack-foods, soups, breakfast cereals, muesli, candies, tabs, cookies, biscuits, crackers, and dairy products.

17. The method of Claim 15 wherein the temperature change is measured at predetermined time intervals for at least fifteen minutes after ingestion of the compound by the subj ect.

18. The method of Claim 17 wherein the predetermined time intervals comprise at least one of (i) five minutes after ingestion, (ii) ten minutes after ingestion, or (iii) fifteen minutes after ingestion.


**Patentansprüche**

1.  Verfahren des Identifizierens einer Verbindung, die die Fettoxidation stimuliert, umfassend:

    Messen einer ersten Temperatur eines Bereichs eines Gesichts eines Subjekts mit einer Thermografievorrichtung, bevor das Subjekt eine Testverbindung einnimmt;

Messen einer zweiten Temperatur des Bereichs des Gesichts des Subjekts mit der Thermografievorrichtung, nachdem die Testverbindung eingenommen ist; und

Klassifizieren der Testverbindung als eine Verbindung, die die Fettoxidation stimuliert, oder eine Verbindung, die keine Fettoxidation stimuliert, mindestens teilweise basierend auf einer Differenz zwischen der zweiten Temperatur und der ersten Temperatur.

2.  Verfahren nach Anspruch 1, wobei der Bereich eine Nase des Subjekts umfasst.

3.  Verfahren nach Anspruch 1, wobei die Thermografievorrichtung eine Infrarotkamera ist.

4.  Verfahren nach Anspruch 1, wobei die erste Temperatur Teil einer ersten Vielzahl von Temperaturen ist, die in ersten vorbestimmten Intervallen über einen ersten vorbestimmten Zeitraum vor der Einnahme der Testverbindung durch das Subjekt erhalten werden, und das Klassifizieren der Testverbindung mindestens teilweise auf einer Differenz zwischen der zweiten Temperatur und einem Durchschnitt der ersten Vielzahl von Temperaturen basiert.

5.  Verfahren nach Anspruch 4, wobei der erste vorbestimmte Zeitraum mindestens dreißig Minuten vor der Einnahme der Testverbindung durch das Subjekt beginnt und bei der Einnahme der Testverbindung durch das Subjekt endet.

6.  Verfahren nach Anspruch 4, wobei die ersten vorbestimmten Intervalle etwa eine Sekunde voneinander entfernt sind.

7.  Verfahren nach Anspruch 1, wobei die zweite Temperatur Teil einer zweiten Vielzahl von Temperaturen ist, die in zweiten vorbestimmten Intervallen über einen zweiten vorbestimmten Zeitraum nach der Einnahme der Testverbindung durch das Subjekt erhalten werden, und das Klassifizieren der Testverbindung mindestens teilweise auf einer Differenz zwischen jeder der zweiten Vielzahl von Temperaturen und der ersten Temperatur basiert.

8.  Verfahren nach Anspruch 7, wobei der zweite vorbestimmte Zeitraum bei der Einnahme der Testverbindung durch das Subjekt beginnt und mindestens fünfzehn Minuten nach dem Verabreichen der Testverbindung endet.

9.  Verfahren nach Anspruch 7, wobei die zweiten vorbestimmten Intervalle etwa eine Sekunde voneinander entfernt sind.

10. Verfahren nach Anspruch 7, umfassend das Bestimmen, für mindestens eine der zweiten Vielzahl von Temperaturen, einer Fläche unter der Kurve (AUC) basierend auf der Differenz in Abhängigkeit von der Zeit, wobei das Klassifizieren der Testverbindung mindestens teilweise auf der AUC basiert.

11. Verfahren nach Anspruch 1, wobei die erste Temperatur Teil einer ersten Vielzahl von Temperaturen ist, die in ersten vorbestimmten Intervallen über einen ersten vorbestimmten Zeitraum vor der Einnahme der Testverbindung durch das Subjekt erhalten werden, die zweite Temperatur Teil einer zweiten Vielzahl von Temperaturen ist, die in zweiten vorbestimmten Intervallen über einen zweiten vorbestimmten Zeitraum nach der Einnahme der Testverbindung durch das Subjekt erhalten werden, und das Klassifizieren der Testverbindung mindestens teilweise auf einer Differenz zwischen jeder der zweiten Vielzahl von Temperaturen und einem Durchschnitt der ersten Vielzahl von Temperaturen basiert.

12. Verfahren nach Anspruch 11, umfassend das Bestimmen, für mindestens eine der zweiten Vielzahl von Temperaturen, einer Fläche unter der Kurve (AUC) basierend auf der Differenz in Abhängigkeit von der Zeit, wobei das Klassifizieren der Testverbindung mindestens teilweise auf der AUC basiert.

13. Verfahren nach Anspruch 1, umfassend das Verabreichen der Testverbindung an das Subjekt.

14. Verfahren nach Anspruch 1, wobei die Testverbindung ohne das Verwenden indirekter Kalorimetrie klassifiziert wird.

15. Verfahren, umfassend:

Messen, mit einer Thermografievorrichtung, einer Temperaturänderung in einem Bereich eines Gesichts eines Subjekts, das eine Verbindung eingenommen hat;

Bestimmen, ob die Verbindung die Fettoxidation stimuliert, mindestens teilweise basierend auf der Temperaturänderung; und

Einbringen der Verbindung in ein Nahrungsmittelprodukt, wenn die Verbindung die Fettoxidation stimuliert.

**16.** Verfahren nach Anspruch 15, wobei das Nahrungsmittelprodukt ausgewählt ist aus der Gruppe bestehend aus Getränken, Backprodukten, Getreideriegeln, Snack-Nahrungsmitteln, Suppen, Frühstückszerealien, Müsli, Bonbons, Tabletten, Keksen, Biskuits, Crackern und Milchprodukten.

**17.** Verfahren nach Anspruch 15, wobei die Temperaturänderung in vorbestimmten Zeitintervallen für mindestens fünfzehn Minuten nach der Einnahme der Verbindung durch das Subjekt gemessen wird.

**18.** Verfahren nach Anspruch 17, wobei die vorbestimmten Zeitintervalle mindestens eines von (i) fünf Minuten nach der Einnahme, (ii) zehn Minuten nach der Einnahme oder (iii) fünfzehn Minuten nach der Einnahme umfassen.

**Revendications**

**1.** Procédé d'identification d'un composé qui stimule l'oxydation de la graisse, comprenant :

la mesure d'une première température d'une région d'un visage d'un sujet avec un dispositif de thermographie avant que le sujet ingère un composé d'essai ;
la mesure d'une seconde température de la région du visage du sujet avec le dispositif de thermographie après que le composé d'essai a été ingéré ; et
la classification du composé d'essai en tant que composé qui stimule l'oxydation de la graisse ou composé qui ne stimule pas l'oxydation de la graisse basée au moins partiellement sur une différence entre la seconde température et la première température.

**2.** Procédé selon la revendication 1 dans lequel la région comprend un nez du sujet.

**3.** Procédé selon la revendication 1 dans lequel le dispositif de thermographie est une caméra infrarouge.

**4.** Procédé selon la revendication 1 dans lequel la première température fait partie d'une première pluralité de températures obtenues dans des premiers intervalles prédéterminés sur un premier laps de temps prédéterminé avant l'ingestion du composé d'essai par le sujet, et la classification du composé d'essai est basée au moins partiellement sur une différence entre la seconde température et une moyenne de la première pluralité de températures.

**5.** Procédé selon la revendication 4 dans lequel le premier laps de temps prédéterminé commence au moins trente minutes avant l'ingestion du composé d'essai par le sujet et se termine à l'ingestion du composé d'essai par le sujet.

**6.** Procédé selon la revendication 4 dans lequel les premiers intervalles prédéterminés sont espacés d'environ une seconde les uns des autres.

**7.** Procédé selon la revendication 1 dans lequel la seconde température fait partie d'une seconde pluralité de températures obtenues dans des seconds intervalles prédéterminés sur un second laps de temps prédéterminé après l'ingestion du composé d'essai par le sujet, et la classification du composé d'essai est basée au moins partiellement sur une différence entre chacune de la seconde pluralité de températures et la première température.

**8.** Procédé selon la revendication 7 dans lequel le second laps de temps prédéterminé commence à l'ingestion du composé d'essai par le sujet et se termine au moins quinze minutes après l'administration du composé d'essai.

**9.** Procédé selon la revendication 7 dans lequel les seconds intervalles prédéterminés sont espacés d'environ une seconde les uns des autres.

**10.** Procédé selon la revendication 7 comprenant la détermination, pour au moins l'une de la seconde pluralité de températures, d'une aire sous la courbe (AUC) basée sur la différence en fonction du temps, dans lequel la classification du composé d'essai est basée au moins partiellement sur l'AUC.

**11.** Procédé selon la revendication 1 dans lequel la première température fait partie d'une première pluralité de températures obtenues dans des premiers intervalles prédéterminés sur un premier laps de temps prédéterminé avant l'ingestion du composé d'essai par le sujet, la seconde température fait partie d'une seconde pluralité de températures obtenues dans des seconds intervalles prédéterminés sur un second laps de temps prédéterminé après l'ingestion du composé d'essai par le sujet, et la classification du composé d'essai est basée au moins partiellement sur une différence entre chacune de la seconde pluralité de températures et une moyenne de la première pluralité de températures.

12. Procédé selon la revendication 11 comprenant la détermination, pour au moins l'une de la seconde pluralité de températures, d'une aire sous la courbe (AUC) basée sur la différence en fonction du temps, dans lequel la classification du composé d'essai est basée au moins partiellement sur l'AUC.

13. Procédé selon la revendication 1 comprenant l'administration du composé d'essai au sujet.

14. Procédé selon la revendication 1 dans lequel le composé d'essai est classifié sans l'utilisation de la calorimétrie indirecte.

15. Procédé comprenant :

la mesure d'un changement de température dans une région d'un visage d'un sujet qui a ingéré un composé, avec un dispositif de thermographie ;
le fait de déterminer si le composé stimule l'oxydation de la graisse basé au moins partiellement sur le changement de température ; et
l'incorporation du composé dans un produit alimentaire si le composé stimule l'oxydation de la graisse.

16. Procédé selon la revendication 15 dans lequel le produit alimentaire est choisi dans le groupe constitué des boissons, des produits cuits, des barres de céréales, des aliments à grignoter, des soupes, des céréales pour le petitdéjeuner, du muesli, des bonbons, des comprimés, des biscuits sucrés, des biscuits, des biscuits salés, et des produits laitiers.

17. Procédé selon la revendication 15 dans lequel le changement de température est mesuré à des intervalles de temps prédéterminés pendant au moins quinze minutes après l'ingestion du composé par le sujet.

18. Procédé selon la revendication 17 dans lequel les intervalles de temps prédéterminés comprennent au moins l'un de (i) cinq minutes après l'ingestion, (ii) dix minutes après l'ingestion, ou (iii) quinze minutes après l'ingestion.

| Basal 30min | | Test 90min | |

Sample ingestion in
200ml of tomato
juice + a slice
of bread

Sample evaluation

Gut comfort evaluation

Recordings:
Session A: Facial T°C, heart rate variability
Session B: EGG, $O_2$ consumption and $CO_2$ production, blood pressure

## FIG. 1A

Z1
Z5
Z7
Z3
Z6
Z8
Z4
Z2
Z11
Z9
Z10
Z12

## FIG. 1B

FIG. 2

FIG. 3A

FIG. 3B

FIG. 3C

FIG. 3D

FIG. 3E

FIG. 3F

FIG. 4A

FIG. 4B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LUDY et al.** The effects of hedonically acceptable red pepper doses on thermogenesis and appetite. *Physiol. Behav.,* 01 March 2011, vol. 102 (3-4), 251-8
- **GREGERSEN et al.** Acute effects of mustard, horseradish, black pepper and ginger on energy expenditure, appetite, ad libitum energy intake and energy balance in human subjects. *Br. J. Nutr.,* July 2012, vol. 5, 1-8
- **A. T. CLARK ; J. S. MANGAT ; S. S. TAY ; Y. KING ; C. J. MONK ; P. A. WHITE ; P. W. EWAN.** Facial thermography is a sensitive and specific method for assessing food challenge outcome. *ALLERGY,* July 2007, vol. 62 (7), 744-749
- Dietary Reference Intakes for Energy, Carbohydrate, Fiber, Fat, Fatty Acids, Cholesterol, Protein, and Amino Acids (Macronutrients). National Academies Press, 2002, 633-645